# EUROPEAN PATENT SPECIFICATION

(11) **EP 3 000 402 B1**
(45) Date of publication and mention of the grant of the patent: **06.03.2019**
(21) Application number: 14841522.7
(22) Date of filing: 19.05.2014
(51) Int. Cl.: A61B 10/02, A61B 1/00, A61B 17/34

(54) **PUNCTURE TREATMENT INSTRUMENT FOR ENDOSCOPE**
PUNKTIONSBEHANDLUNGSINSTRUMENT FÜR ENDOSKOP
INSTRUMENT DE TRAITEMENT DE PONCTION POUR ENDOSCOPE

(30) Priority: 04.09.2013 JP 2013183287
(43) Date of publication of application: 30.03.2016
(73) Proprietor: Olympus Corporation, Tokyo 192-8507 (JP)
(72) Inventor: HATAKEYAMA, Tomoyuki, Hachioji-shi Tokyo 192-8507 (JP); NISHINA, Kenichi, Hachioji-shi Tokyo 192-8507 (JP)
(74) Representative: Schicker, Silvia
(86) International application number: PCT/JP2014/063212
(87) International publication number: WO 2015/033618

(56) References cited:
- WO-A1-2007/010796
- WO-A1-2008/101286
- JP-A- H07 289 636
- JP-A- 2003 153 911
- JP-A- 2010 094 367
- JP-A- 2010 252 967
- US-A1- 2010 222 745
- US-A1- 2011 313 246

## Description

### Technical Field

The present invention relates to a puncture treatment instrument for endoscope inserted and used in a treatment instrument insertion channel of an endoscope.

### Background Art

An example of a technique of performing a treatment, such as collection of tissue and injection, in a subject, includes a method of bringing a puncture treatment instrument for endoscope into the subject through a treatment instrument insertion channel of an endoscope, the method disclosed in Japanese Patent Application Laid-Open Publication No. 2001-37775.

A distal end of the puncture treatment instrument for endoscope is sharp, and contact with other objects needs to be avoided. The puncture treatment instrument for endoscope has a long shape with a small diameter, and the puncture treatment instrument for endoscope is bent by its own weight. Therefore, operation of inserting the puncture treatment instrument for endoscope into an opening portion of the treatment instrument insertion channel provided in the endoscope and handling after removal from the treatment instrument channel are difficult.

Document US 2010/222745 A1 relates to a cannula, such as an IV catheter introducer needle, that has a bi-directionally engageable crimp feature. The feature comprises a maximum outer diameter that is greater than an outer diameter of the cannula. Additionally, the feature comprises a proximal side and a distal side. The larger outer diameter of the proximal side can act as a proximal engagement that prevents the cannula from being entirely extracted in a proximal direction from a cannula shield. The distal side of the feature can comprise a notch that acts as a distal engagement, which prevents the cannula from reemerging distally from the shield, once the cannula has been pulled into the shield. The notch can include an engagement surface that extends laterally past the cannula's outer diameter. The engagement surface can be configured to run substantially perpendicular to the cannula's longitudinal axis.

An object of the present invention is to facilitate handling of a puncture treatment instrument for endoscope.

### Disclosure of Invention

### Means for Solving the Problem

A puncture treatment instrument for endoscope according to an aspect of the present invention includes: a concave portion in a concave shape with an opening on a distal end side and a bottom surface on a proximal end side, a depth of the concave
portion from a distal end to the bottom surface being a first distance; a holding portion with a distal end side adjacent to a proximal end of the concave portion, the holding portion being made of an elastic material that allows insertion of a needle; a puncture needle with a distal end stuck into the holding portion and a proximal end exposed from the holding portion, a full length of the puncture needle being longer than a distance from a proximal end of the holding portion to a distal end of the concave portion; and a barb formed within the first distance from the distal end on a surface of the puncture needle, the barb stopping retraction of the puncture needle by being caught by the bottom surface of the concave portion when the puncture needle is retracted after the puncture needle is advanced to project into the concave portion from the holding portion.

A puncture treatment instrument for endoscope according to an illustrative example includes: a concave portion with an opening on a distal end side and a bottom surface on a proximal end side; a holding portion adjacent to a proximal end of the concave portion, the holding portion including a needle insertion path that allows advancement and retraction of a needle; a puncture needle with a full length longer than a distance from a proximal end of the holding portion to a distal end of the concave portion; and a string portion linking a predetermined position of the puncture needle and the holding portion, a length of the string portion in a tense state being a length such that a distal end of the puncture needle is arranged in the concave portion or in the holding portion.

### Brief Description of the Drawings

Fig. 1 is a side view of a puncture treatment instrument for endoscope according to a first embodiment;
Fig. 2 is a diagram showing an example of configuration of an endoscope;
Fig. 3 is a cross-sectional view of a distal end portion of a puncture needle and a slider according to the first embodiment;
Fig. 4 is a cross-sectional view showing a state that a locking portion of a barb is in contact with a distal end surface of a holding portion according to the first embodiment;
Fig. 5 is a cross-sectional view showing a state that a fitting portion is fitted to a treatment instrument introducing port according to the first embodiment;
Fig. 6 is a cross-sectional view describing an action of the first embodiment;
Fig. 7 is a cross-sectional view describing an action of the first embodiment;
Fig. 8 is a cross-sectional view showing a first modification of the first embodiment;
Fig. 9 is a cross-sectional view showing a second modification of the first embodiment;
Fig. 10 is a cross-sectional view of the distal end portion of the puncture needle and the slider according to a first illustrative example;
Fig. 11 is a side view of the puncture treatment instrument for endoscope according to a second embodiment;
Fig. 12 is a cross-sectional view of the distal end portion of the puncture needle and the slider according to the second embodiment;
Fig. 13 is a cross-sectional view showing a modification of the second embodiment;
Fig. 14 is a cross-sectional view of the distal end portion of the puncture needle and the slider according to a second illustrative example;
Fig. 15 is a cross-sectional view showing a modification of the second illustrative example;
Fig. 16 is a cross-sectional view of the distal end portion of the puncture needle and the slider according to a third embodiment; and
Fig. 17 is a cross-sectional view showing a modification of the third embodiment.

### Best Mode for Carrying Out the Invention

Hereinafter, a best mode of the present invention will be described with reference to the drawings. Note that in each drawing used in the following description, scaling varies from one constituent element from another to make the size of each constituent element recognizable on the drawings. The present invention is not limited only to quantities of the constituent elements, shapes of the constituent elements, ratios of sizes of the constituent elements, and relative positional relationships between the constituent elements described in the drawings.

### (First Embodiment)

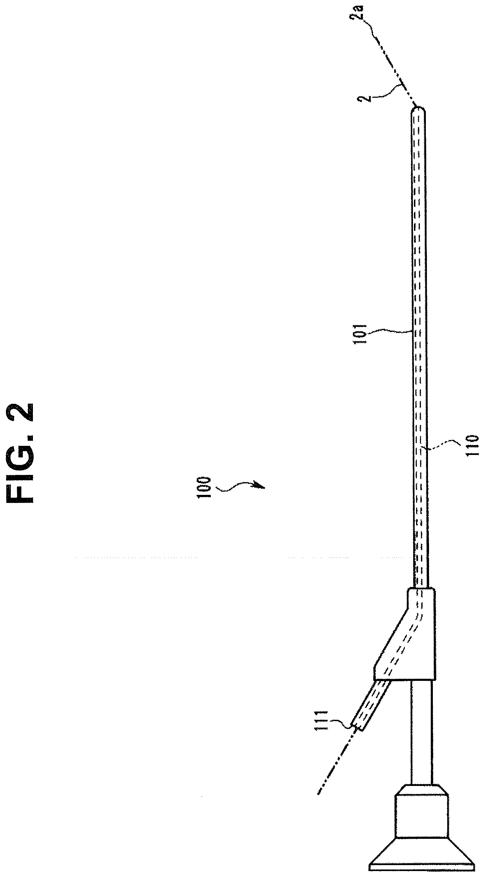

Hereinafter, an example of an embodiment of a puncture treatment instrument for endoscope according to the present invention will be described. A puncture treatment instrument for endoscope 1 includes a puncture needle 2, and the puncture treatment instrument for endoscope 1 is inserted and used in a treatment instrument insertion channel 110 provided in an endoscope 100 as illustrated in Fig. 2. Note that although the endoscope 100 illustrated in Fig. 2 is a so-called rigid endoscope in
which an insertion portion 101 introduced into a subject is rigid, the endoscope 100 may be in a form of a so-called flexible endoscope in which the insertion portion 101 is flexible. The endoscope 100 may also be a so-called ultrasound endoscope including an ultrasound transducer.

The endoscope 100 includes a cylindrical treatment instrument introducing port 111 linked to the treatment instrument insertion channel 110. That is, a proximal end of the treatment instrument insertion channel 110 opens at the cylindrical treatment instrument introducing port 111. The treatment instrument insertion channel 110 also opens at a distal end portion of the insertion portion 101. As indicated by an alternate long and two short dashes line in Fig. 2, the puncture needle 2 of the puncture treatment instrument for endoscope 1 inserted into the treatment instrument insertion channel 110 from the treatment instrument introducing port 111 projects from the distal end portion of the insertion portion 101.

A type of the puncture treatment instrument for endoscope 1 according to the present invention is, for example, a biopsy needle for collecting tissue in a subject or an injection needle for performing a therapeutic treatment, such as injection, in a subject and is not particularly limited.

The puncture treatment instrument for endoscope 1 includes the puncture needle 2, an operation portion 3, and a slider 10. The puncture needle 2 is an elongated part that can be inserted into the treatment instrument insertion channel 110 of the endoscope 100. A distal end portion 2a of the puncture needle 2 is provided with a barb 4 described in detail later.

A needle insertion path 12 that is a hole portion for inserting the puncture needle 2 is formed in the slider 10, and this will be described in detail later. In a state that the puncture treatment instrument for endoscope 1 is not used, the slider 10 is installed at a position surrounding the distal end portion 2a of the puncture needle 2 inserted into the needle insertion path 12.

The operation portion 3 is a part connected to a proximal end 2b of the puncture needle 2. The operation portion 3 is a part held by a user when the user uses the puncture treatment instrument for endoscope 1 and is a part for executing operation of advancing, retracting, and rotating the puncture needle 2 in the treatment instrument insertion channel 110 or operation of controlling a mechanism provided in the puncture needle 2.

When the puncture treatment instrument for endoscope 1 is, for example, a biopsy needle including double needles, an inner needle and an outer needle, the operation portion 3 includes a knob for advancing and retracting the outer needle relative to the inner needle. When the puncture treatment instrument for endoscope 1 is, for example, an injection needle, an opening portion for connecting a syringe is provided.

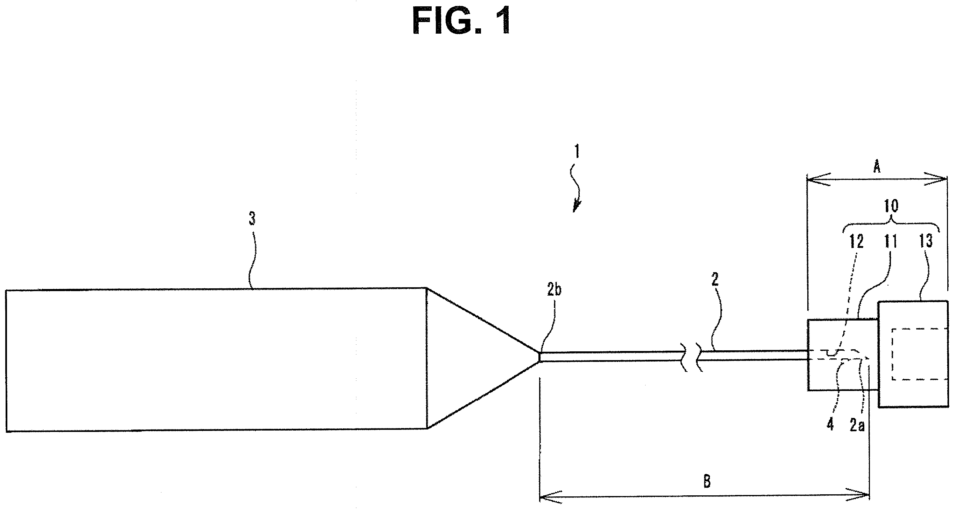

The puncture treatment instrument for endoscope 1 of the present embodiment illustrated in Fig. 1 is in a form of a biopsy needle, for example. That is, the puncture needle 2 is a hollow needle tube with an outer diameter that allows insertion into the treatment instrument insertion channel 110 of the endoscope 100.

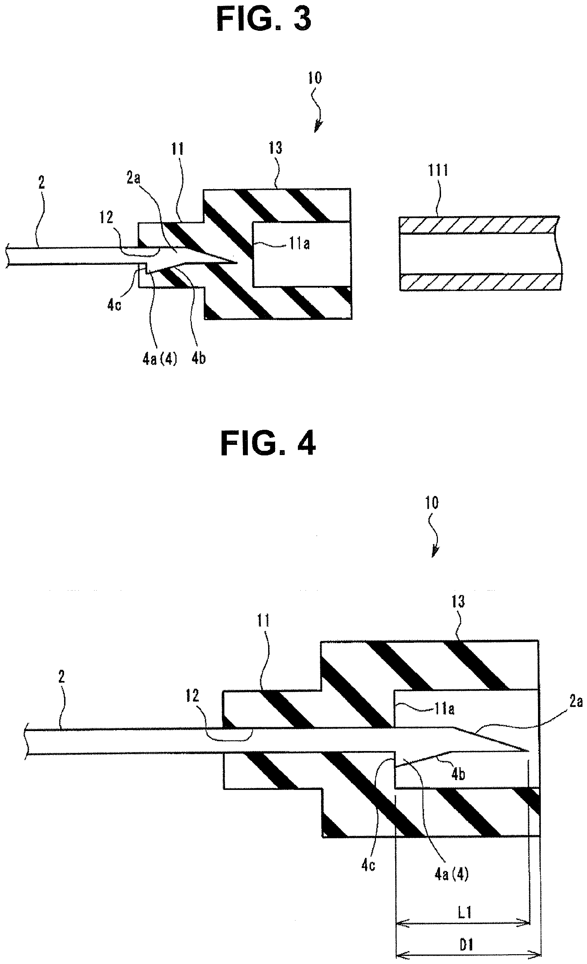

As shown in Fig. 3, the distal end of the puncture needle 2 is sharp, and a predetermined part in the subject can be punctured by the distal end portion 2a through the treatment instrument insertion channel 110 to collect tissue of the part.

The slider 10 includes a holding portion 11, the needle insertion path 12, and a concave portion 13. The concave portion 13 is a concave part with an opening on a distal end side and a bottom surface on a proximal end side. A distal end side of the holding portion 11 is adjacent to a proximal end of the concave portion 13, and the holding portion 11 is made of an elastic material that allows insertion of the puncture needle 2. A full length of the slider 10, that is, a distance from a distal end of the concave portion 13 to a proximal end of the holding portion 11 (indicated by A in Fig. 1), is shorter than a full length of the puncture needle 2 (indicated by B in Fig. 1).

More specifically, the holding portion 11 is a part provided with the needle insertion path 12 for inserting the puncture needle 2. The needle insertion path 12 is a hole portion that opens at least on the proximal end side of the holding portion 11. The needle insertion path 12 may be formed to penetrate through the holding portion 11 or may be formed not to penetrate through the holding portion 11 as in the present embodiment illustrated in Fig. 3.

When the needle insertion path 12 is formed not to penetrate through the holding portion 11 as in the present embodiment, the slider 10 is made of a member that allows the distal end portion 2a of the puncture needle 2 to penetrate. For example, the slider 10 is made of an elastically deformable material, such as rubber, in the present embodiment.

An inner diameter of the needle insertion path 12 is smaller than the outer diameter of the puncture needle 2 in a state that the puncture needle 2 is not inserted. Therefore, when the puncture needle 2 is inserted into the needle insertion path 12, the inner diameter of the needle insertion path 12 is expanded, and an outer circumferential face of the puncture needle 2 and an inner circumferential face of the needle insertion path 12 come into close contact with each other. Note that in the state that the puncture needle 2 is inserted into the needle insertion path 12, a gap may exist between the outer circumferential face of the puncture needle 2 and the inner circumferential face of the needle insertion path 12.

As described, the slider 10 is made of an elastically deformable material, such as rubber, and the distal end of the puncture needle 2 has a needle-like sharp shape. Therefore, when the puncture needle 2 is pushed into the slider 10 to move the puncture needle 2 in a distal end direction relative to the slider 10 from the state that the puncture needle 2 is inserted into the needle insertion path 12, the puncture needle 2 penetrates through the holding portion 11.

Note that the needle insertion path 12 may not be a hole portion formed in advance by drilling, molding, or the like in the holding portion 11, and the needle insertion path 12 may be a hole portion formed in the holding portion 11 by sticking the puncture needle 2 into the holding portion 11.

Here, the barb 4 is provided in the distal end portion 2a of the puncture needle 2 inserted into the needle insertion path 12. The barb 4 is a part that restricts a range of the movement of the slider 10 relative to the puncture needle 2.

The barb 4 is formed by a convex portion 4a projecting toward the outside in a radial direction from part of a circumference surface of the distal end portion 2a. An inclined plane 4b inclined toward the outside in the radial direction from the distal end side to the proximal end side is formed on the distal end side of the convex portion 4a.

On the other hand, a locking portion 4c is provided on a proximal end side of the convex portion 4a, the locking portion 4c restricting further relative movement of the puncture needle 2 in the proximal end direction by being caught by the distal end surface 11a of the holding portion 11 when the puncture needle 2 is moved in the proximal end direction relative to the slider 10 after the distal end portion 2a penetrates through the holding portion 11 as shown in Fig. 4.

So to speak, the barb 4 permits the distal end portion 2a of the puncture needle 2 to enter the needle insertion path 12 from the proximal end side and permits the relative movement of the distal end portion 2a in the distal end direction in the needle insertion path 12. The barb 4 also prohibits relative movement of the distal end portion 2a in the proximal end direction in the needle insertion path 12 and prohibits the distal end portion 2a from entering the needle insertion path 12 from the distal end side. The configuration of the barb 4 is similar to a configuration generally called a ratchet mechanism.

In other words, the convex portion 4a that is the barb 4 includes the locking portion 4c with an effect similar to a part called a hook or the like provided at a distal end of a fishhook. The barb 4 generates large resistance force when the puncture needle 2 is moved in the proximal end direction relative to the slider 10 to pull out the puncture needle 2 from the needle insertion path 12.

The concave portion 13 is a part provided closer to the distal end with respect to the distal end surface 11a of the holding portion 11 and fitted to the treatment instrument introducing port 111 provided in the endoscope 100. The concave portion 13 is fitted to the treatment instrument introducing port 111 to position the needle insertion path 12 provided in the holding portion 11, at a location substantially on the same axis as the treatment instrument introducing port 111.

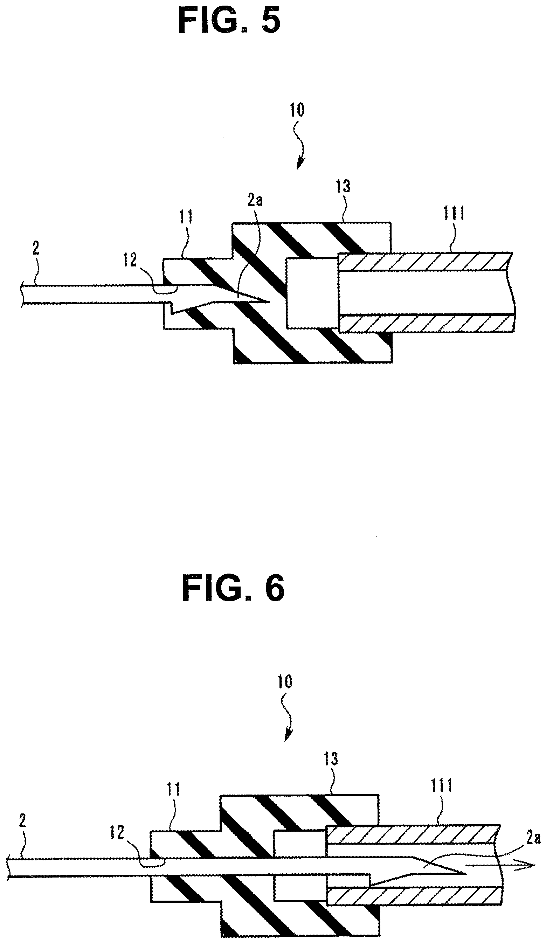

In the present embodiment, the concave portion 13 is a cylindrical part projecting toward the distal end side with respect to the distal end surface 11a of the holding portion 11 and fitted to the outer circumference of the treatment instrument introducing port 111, for example. The cylindrical concave portion 13 is installed substantially on the same axis as the needle insertion path 12. Therefore, as shown in Fig. 5, the concave portion 13 is fitted to the treatment instrument introducing port 111 to position the needle insertion path 12 provided in the holding portion 11, substantially on the same axis as the treatment instrument introducing port 111. In other words, the concave portion 13 is fitted to the treatment instrument introducing port 111 to position the distal end portion 2a of the puncture needle 2 substantially on the same axis as the treatment instrument introducing port 111.

In the present embodiment, the concave portion 13 has an inner diameter smaller than the outer diameter of the treatment instrument introducing port 111 in the state that the concave portion 13 is not fitted to the treatment instrument introducing port 111. Therefore, when the concave portion 13 is fitted to the treatment instrument introducing port 111 as shown in Fig. 5, the inner diameter of the concave portion 13 is expanded, and the outer circumferential face of the treatment instrument introducing port 111 and the inner circumferential face of the concave portion 13 come into close contact with each other. Note that a gap may exist between the inner circumferential face of the concave portion 13 and the outer circumferential face of the treatment instrument introducing port 111 in the state that the concave portion 13 and the treatment instrument introducing port 111 are fitted.

As shown in Fig. 4, the concave portion 13 projects in the distal end direction with respect to the distal end of the puncture needle 2 in the state that the locking portion 4c provided in the distal end portion 2a of the puncture needle 2 is in contact with the distal end surface 11a of the holding portion 11. Specifically, a first distance D1 from the distal end surface 11a of the holding portion 11 to the distal end surface 13a of the concave portion 13 is longer than a distance L1 from the locking portion 4c to the distal end of the puncture needle 2. The first distance D1 is equal to a depth from the distal end to the bottom surface of the concave portion 13, and the locking portion 4c of the barb 4 is formed at a position within the first distance D1 from the distal end, on the surface of the puncture needle 2.

Therefore, the outside of the distal end portion 2a of the puncture needle 2 in the radial direction is covered by the cylindrical concave portion 13 in the state that the movement of the puncture needle 2 in the proximal end direction relative to the slider 10 is restricted by the barb 4 (state illustrated in Fig. 4).

In other words, the distal end portion 2a of the puncture needle 2 is housed in the concave portion 13 in the state that the movement of the puncture needle 2 in the proximal end direction relative to the slider 10 is restricted by the barb 4.

In the present embodiment described above, the distal end portion 2a of the puncture needle 2 falls within the needle insertion path 12 of the slider 10 as shown in Fig. 3 in a state that the puncture treatment instrument for endoscope 1 is not used at all, that is, a state before the puncture needle 2 is inserted into the treatment instrument insertion channel 110 of the endoscope 100. In this state, the puncture needle 2 does not penetrate through the holding portion 11, and the distal end portion 2a of the puncture needle 2 is covered by the holding portion 11.

To use the puncture treatment instrument for endoscope 1, the user holds the slider 10 to fit the concave portion 13 to the treatment instrument introducing port 111 of the endoscope 100 as shown in Fig. 5. The operation of fitting the concave portion 13 to the treatment instrument introducing port 111 can be more easily carried out than operation of inserting a naked distal end portion 2a of the puncture needle 2 with a smaller diameter into the treatment instrument introducing port 111.

The concave portion 13 is fitted to the treatment instrument introducing port 111 of the endoscope 100, and the distal end portion 2a of the puncture needle 2 is positioned substantially on the same axis as the treatment instrument introducing port 111. Therefore, if the puncture needle 2 is pushed in the distal end direction, the puncture needle 2 penetrates through the holding portion 11 and enters inside the treatment instrument introducing port 111, that is, inside the treatment instrument insertion channel 110, as shown in Fig. 6.

Here, the outer circumferential face of the treatment instrument introducing port 111 and the inner circumferential face of the concave portion 13 come into close contact with each other, and the outer circumferential face of the puncture needle 2 and the inner circumferential face of the needle insertion path 12 come into close contact with each other. This can prevent leakage of a perfusate from the treatment instrument introducing port 111.

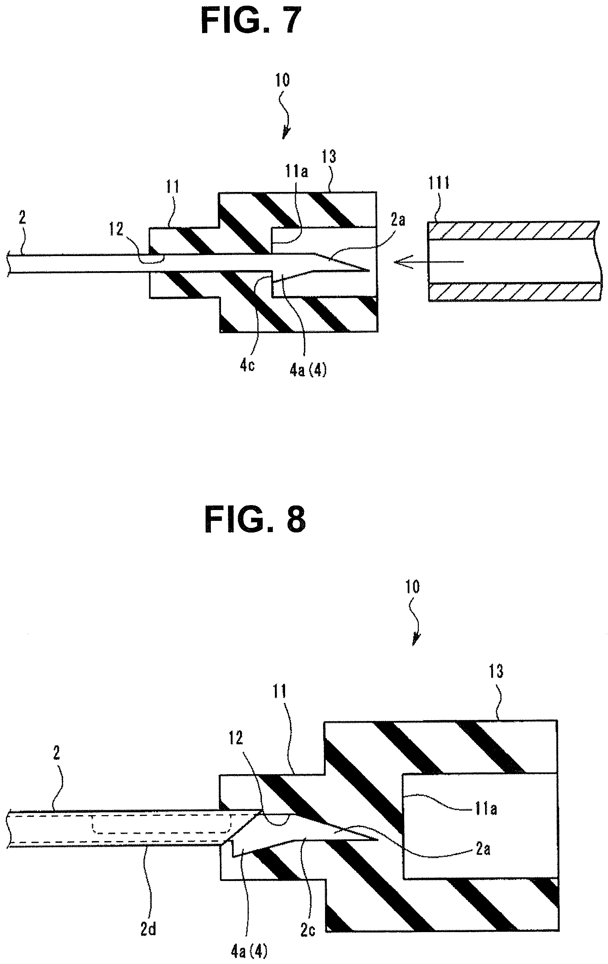

When the puncture needle 2 is removed from the inside of the treatment instrument insertion channel 110 after a target site in the subject is punctured by the distal end portion 2a of the puncture needle 2, the locking portion 4c provided in the distal end portion 2a is caught by the distal end surface 11a of the holding portion 11 (bottom surface of the concave portion 13), and the slider 10 follows the distal end portion 2a and comes off the treatment instrument introducing port 111 as shown in Fig. 7. In this case, the outside of the distal end portion 2a of the puncture needle 2 in the radial direction is covered by the concave portion 13, and the distal end of the puncture needle 2 is inside of the concave portion 13.

As described, the puncture treatment instrument for endoscope 1 of the present embodiment includes the slider 10, and the operation of inserting the puncture needle 2 into the treatment instrument insertion channel 110 can be facilitated.

The puncture treatment instrument for endoscope 1 of the present embodiment includes the barb 4 that prevents the slider 10 from falling out from the distal end portion 2a side of the puncture needle 2, and the slider 10 can be repeatedly used when the puncture needle 2 is inserted again into the treatment instrument insertion channel 110.

The puncture treatment instrument for endoscope 1 of the present embodiment includes the concave portion 13 that covers the distal end portion 2a in the state that the movement of the slider 10 toward the distal end side relative to the puncture needle 2 is restricted by the barb 4. This can prevent the distal end portion 2a of the puncture needle 2 from coming into contact with the user or a surrounding object, not only in the unused state, but also in the state after the removal from the treatment instrument insertion channel 110. Therefore, handling of the puncture treatment instrument for endoscope 1 of the present embodiment is easier than in the conventional techniques in which the puncture needle is naked.

Note that although the puncture treatment instrument for endoscope 1 of the present embodiment is a biopsy needle made of a single needle-like tube, the puncture treatment instrument for endoscope 1 may be formed by double needle-like tubes, an inner needle 2c and an outer needle 2d, as illustrated in a first modification in Fig. 8 when the puncture treatment instrument for endoscope 1 is a biopsy needle. The first modification also attains the same effects as in the embodiment described above.

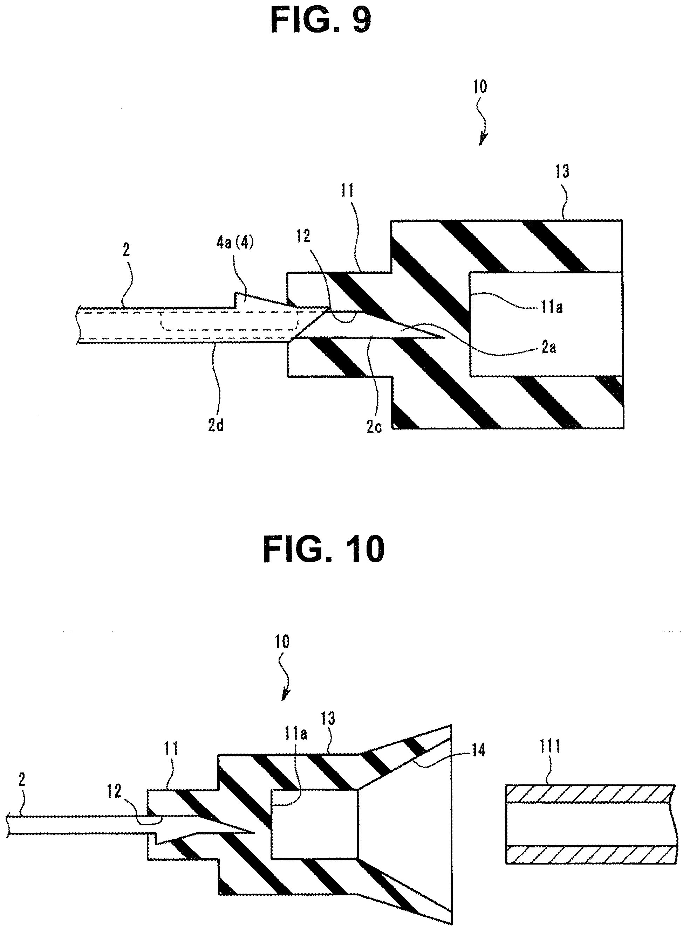

Note that although the barb 4 is provided in the inner needle in the first modification illustrated in Fig. 8, the same effects as in the embodiment described above are also attained even if the barb 4 is provided in the outer needle 2d as illustrated in a second modification in Fig. 9.

### (First illustrative example)

Hereinafter, a first illustrative example will be described. Part of the configuration of the puncture treatment instrument for endoscope 1 of the first illustrative example is different from the first embodiment described above. Therefore, the same constituent elements as in the first embodiment are designated with the same reference signs, and the description will be appropriately omitted.

As shown in Fig. 10, the puncture treatment instrument for endoscope 1 of the first illustrative example is different from the first embodiment in that a guide portion 14 is provided on the distal end side of the concave portion 13 of the slider 10. The guide portion 14 is a part including a tapered inner circumferential face with an inner diameter expanding from the proximal end side to the distal end side. An inner diameter of a proximal end of the guide portion 14 is the same as the inner diameter of the concave portion 13.

In this way, the guide portion 14 with the inner diameter expanding toward the distal end side can be provided at the distal end of the slider 10 to more easily perform the operation of fitting the concave portion 13 to the treatment instrument introducing port 111 than in the first embodiment. The puncture treatment instrument for endoscope 1 of the first illustrative example attains the same effects as in the first embodiment.

### (Second Embodiment)

Hereinafter, a second embodiment of the present invention will be described. A component of the puncture treatment instrument for endoscope 1 of the present embodiment for restricting the movement of the slider 10 relative to the puncture needle 2 is different from that of the first embodiment and the first illustrative example described above. Therefore, the same constituent elements as in the first embodiment and the first illustrative example are designated with the same reference signs, and the description will be appropriately omitted.

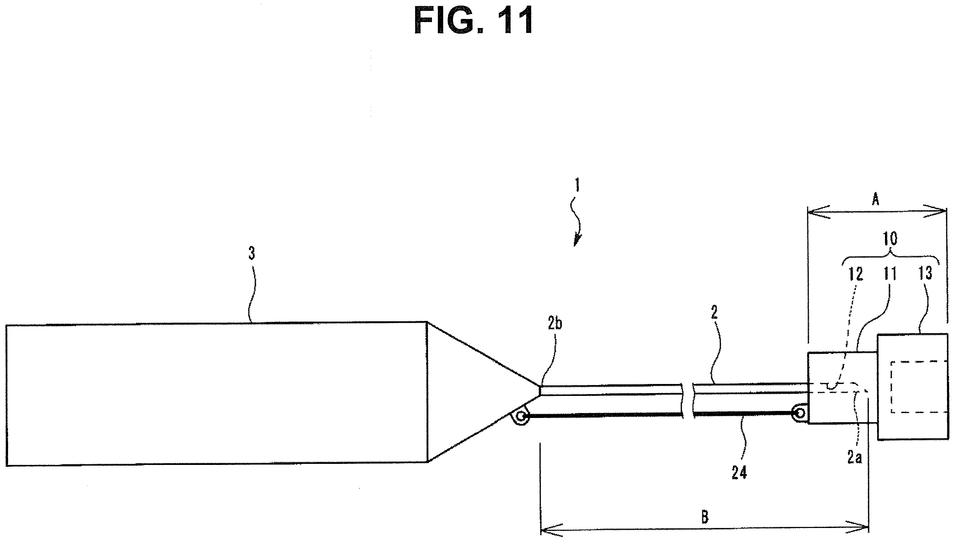

As shown in Fig. 11, the component for restricting the movement of the slider 10 relative to the puncture needle 2 in the present embodiment is a string portion 24 that connects the slider 10 with the operation portion 3 or the proximal end 2b of the puncture needle 2. The string portion 24 is a flexible member with a predetermined length in a tense state after application of tensile force.

The string portion 24 is formed by, for example, a string-like member or a chain-like member. The string portion 24 may be a member with fiber woven in a mesh pattern. For example, if the string portion 24 is a mesh-like member, the string portion 24 can be a tubular mesh member, and the string portion 24 can be installed to surround the puncture needle 2.

In the present embodiment illustrated in Fig. 11, the string portion 24 is a string-like member with a predetermined length. The string portion 24 connects the slider 10 and the operation portion 3 and prevents the slider 10 from falling out from the distal end portion 2a side of the puncture needle 2.

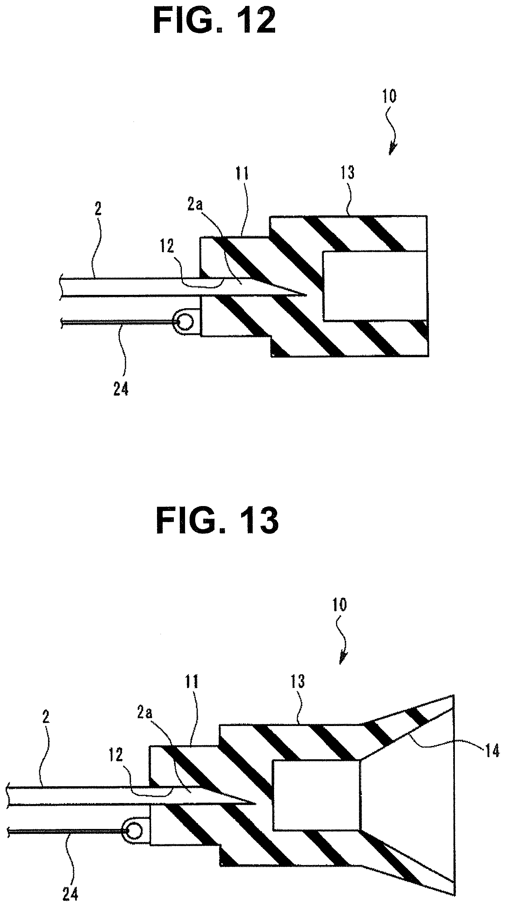

The length of the string portion 24 is set to a length such that the distal end portion 2a of the puncture needle 2 is located in the holding portion 11 or in the concave portion 13 as shown in Fig. 12 in the state that the string portion 24 is tense and in the state that the movement of the slider 10 toward the distal end side relative to the puncture needle 2 is restricted.

The puncture treatment instrument for endoscope 1 of the present embodiment in this way includes the slider 10 as in the first embodiment described above, and the operation of inserting the puncture needle 2 into the treatment instrument insertion channel 110 and handling of the puncture needle 2 after the removal can be facilitated. The puncture treatment instrument for endoscope 1 of the present embodiment also includes the string portion 24 that prevents the slider 10 from falling out from the distal end portion 2a side of the puncture needle 2, and the slider 10 can be repeatedly used when the puncture needle 2 is inserted again into the treatment instrument insertion channel 110.

Compared to the barb 4 formed in the distal end portion 2a of the puncture needle 2 of the first embodiment, the string portion 24 of the present embodiment can be easily formed. The shape of the distal end portion 2a of the puncture needle 2 does not have to be changed in the present embodiment, and application to a conventional apparatus is easier.

Note that as illustrated in a modification in Fig. 13, the guide portion 14 with the inner diameter expanding toward the distal end side may be formed in the distal end portion of the slider 10 as in the first illustrative example.

### (Second illustrative example)

Hereinafter, a second illustrative example will be described. The configuration of the puncture treatment instrument for endoscope 1 of the second illustrative example for restricting the movement of the slider 10 relative to the puncture needle 2 is different from the first embodiment and the first illustrative example described above. The same constituent elements as in the first embodiment and the first illustrative example are designated with the same reference signs, and the description will be appropriately omitted.

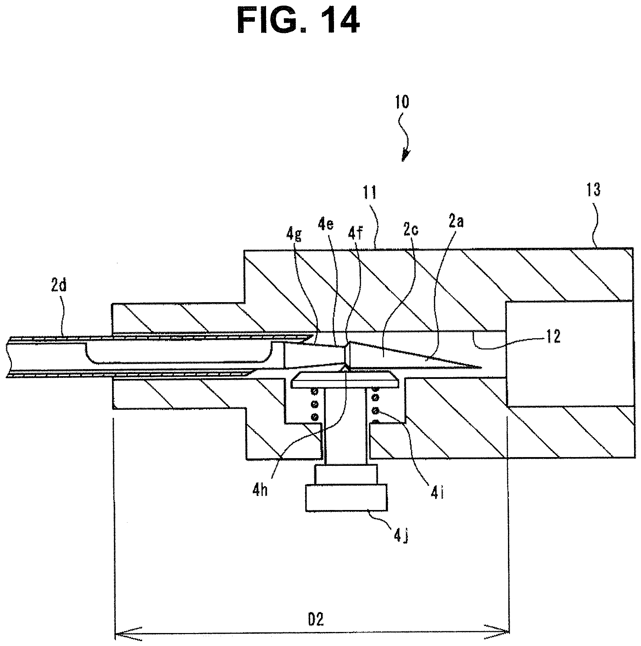

In the second illustrative example illustrated in Fig. 14, a recessed portion 4e provided in the distal end portion 2a of the puncture needle 2 and a pressing portion 4h and an urging spring 4i installed in the slider 10 restrict the movement of the slider 10 relative to the puncture needle 2. In the second illustrative example, the length of the holding portion 11 from the distal end to the proximal end is a second distance
D2. The needle insertion path 12 is a through hole penetrating through the holding portion 11, and the puncture needle 2 can be advanced and retracted inside of the needle insertion path 12. In the second illustrative example, the puncture needle 2 is, for example, a biopsy needle formed by double needle-like tubes, the inner needle 2c and the outer needle 2d.

The recessed portion 4e is a groove-like part in which a cross section carved in the outer circumferential direction on the outer circumference of the distal end portion 2a of the puncture needle 2 is substantially V-shaped. The recessed portion 4e includes: a tapered portion 4g formed on the surface of the puncture needle 2 within the second distance from the distal end, with a diameter decreasing toward the distal end; and a stepped portion 4f formed adjacently on the distal end side of the tapered portion 4g, with a diameter increasing toward the distal end at a steeper slope than the tapered portion 4g. The recessed portion 4e is provided on the outer circumference of the inner needle 2c.

The pressing portion 4h is a member that can project and recess toward the inside of the needle insertion path 12 in the radial direction. The pressing portion 4h is urged by the urging spring 4i toward the inside of the needle insertion path 12 in the radial direction, and the pressing portion 4h engages with the recessed portion 4e provided on the outer circumference of the distal end portion 2a of the puncture needle 2.

The pressing portion 4h is provided with a handle 4j projecting toward the outside in the radial direction. The handle 4j projects outside of the outer circumference of the holding portion 11. The user pulls the handle 4j toward the outside of the holding portion 11, and the pressing portion 4h exits from an internal space of the needle insertion path 12.

In the second illustrative example, the slope of the tapered portion 4g of the recessed portion 4e is moderate in a state that the pressing portion 4h is pressed into the recessed portion 4e by the urging spring 4i, and the puncture needle 2 can be moved toward the distal end side relative to the slider 10. On the other hand, the slope of the stepped portion 4f of the recessed portion 4e is steep in the state that the pressing
portion 4h is pressed into the recessed portion 4e by the urging spring 4i, and large resistance force is generated to prevent the movement of the puncture needle 2 toward the proximal end side relative to the slider 10.

The puncture treatment instrument for endoscope 1 of the second illustrative example includes the slider 10 as in the first embodiment described above, and the operation of inserting the puncture needle 2 into the treatment instrument insertion channel 110 can be facilitated.

As described, the pressing portion 4h is pressed into the recessed portion 4e to prevent the slider 10 from falling out from the distal end portion 2a side of the puncture needle 2 as in the first embodiment. Therefore, handling of the puncture needle 2 after the removal from the treatment instrument insertion channel 110 is also easy. The slider 10 can be repeatedly used when the puncture needle 2 is inserted again into the treatment instrument insertion channel 110.

In the second illustrative example, the handle 4j can be operated to release the engagement of the pressing portion 4h and the recessed portion 4e, and the slider 10 can be easily removed from the puncture needle 2.

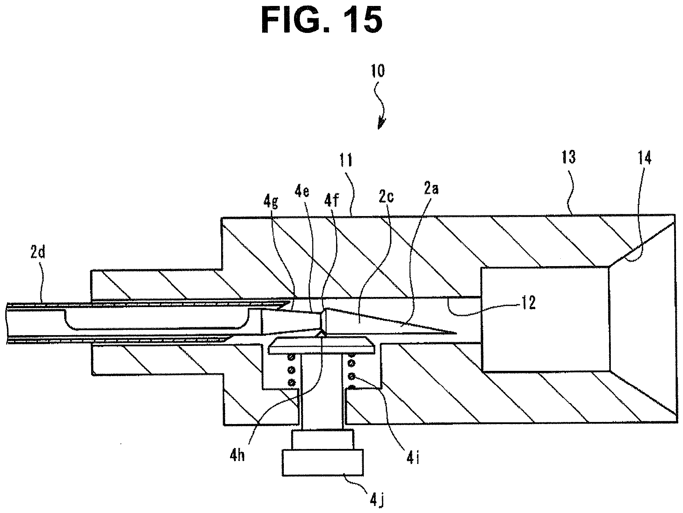

Note that as illustrated in a modification in Fig. 15, the guide portion 14 with the inner diameter expanding toward the distal end side may be formed in the distal end portion of the slider 10 as in the first illustrative example.

### (Third Embodiment)

Hereinafter, a third embodiment of the present invention will be described. In the puncture treatment instrument for endoscope 1 of the present embodiment, the configuration of the concave portion 13 is different from that of the second embodiment described above. The same constituent elements as in the second embodiment are designated with the same reference signs, and the description will be appropriately omitted.

Although the concave portion 13 is fixed to the slider 10 in the second embodiment described above, the concave portion may be able to be removed from the slider 10.

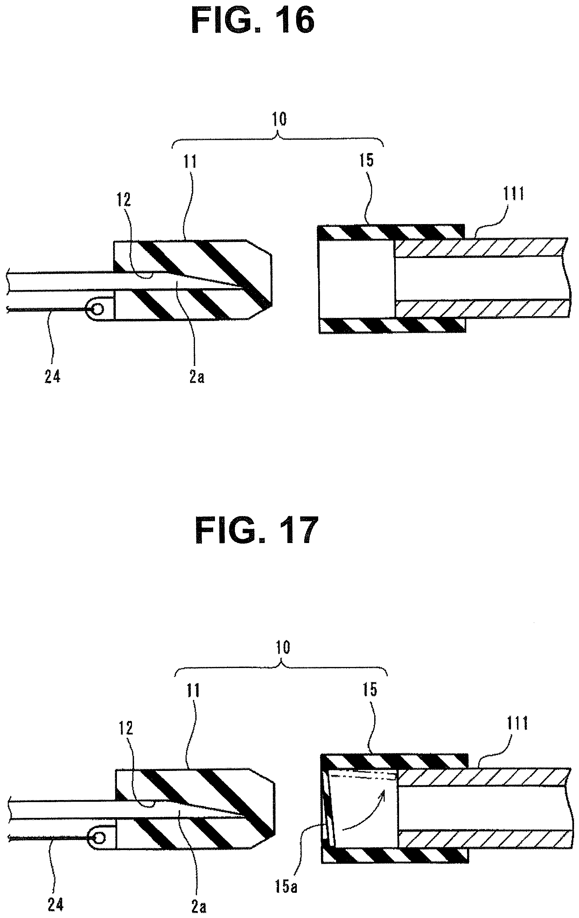

In the present embodiment illustrated in Fig. 16, the concave portion 15 is a cylindrical member fitted to the outer circumference of the treatment instrument introducing port 111 on the distal end side and fitted to the outer circumference of the holding portion 11 on the proximal end side. In the present embodiment, the length of the string portion 24 is set such that the distal end portion 2a of the puncture needle 2 is at a position covered by the holding portion 11 in the state that the string portion 24 is tense and in the state that the movement of the holding portion 11 toward the distal end side relative to the puncture needle 2 is restricted.

To insert the puncture treatment instrument for endoscope 1 into the treatment instrument insertion channel 110 in the present embodiment, a distal end side of the cylindrical concave portion 15 is first fitted to the treatment instrument introducing port 111, and then the holding portion 11 is fitted inside of a proximal end side of the concave portion 15. The operation positions the distal end portion 2a of the puncture needle 2 substantially on the same axis as the treatment instrument introducing port 111.

Therefore, the puncture treatment instrument for endoscope 1 of the present embodiment includes the slider 10 as in the second embodiment described above, and the operation of inserting the puncture needle 2 into the treatment instrument insertion channel 110 and handling of the puncture needle 2 after the removal can be facilitated. The puncture treatment instrument for endoscope 1 of the present embodiment includes the string portion 24 that prevents the slider 10 from falling out from the distal end portion 2a side of the puncture needle 2, and the slider 10 can be repeatedly used when the puncture needle 2 is inserted again into the treatment instrument insertion channel 110.

Note that as illustrated in a modification in Fig. 17, a check valve 15a may be installed in the concave portion 15. The check valve 15a is installed to block a flow of a fluid from the distal end side to the proximal end side of the concave portion 15. The check valve 15a permits the insertion of the holding portion 11 or the puncture needle 2 into the concave portion 15 from the proximal end side of the concave portion 15. The installation of the check valve 15a in the concave portion 15 as in the modification can prevent leakage of a perfusate from the treatment instrument introducing port 111.

Note that the present invention is not limited to the embodiments described above.

## Claims

1. A puncture treatment instrument for endoscope (1) comprising:
a concave portion (13) in a concave shape with an opening on a distal end side and a bottom surface (11a) on a proximal end side, a depth of the concave portion from a distal end to the bottom surface (11a) being a first distance;
a holding portion (11) with a distal end side adjacent to a proximal end of the concave portion (13), the holding portion being made of an elastic material that allows insertion of a needle; and
a puncture needle (2) with a distal end stuck into the holding portion (11) and a proximal end exposed from the holding portion (11), a full length of the puncture needle being longer than a distance from a proximal end of the holding portion (11) to a distal end of the concave portion (13);
**characterized by** further comprising:
a barb (4) formed within the first distance from the distal end on a surface of the puncture needle (2), the barb stopping retraction of the puncture needle (2) by being caught by the bottom surface (11a) of the concave portion (13) when the puncture needle (2) is retracted after the puncture needle is advanced to project into the concave portion (13) from the holding portion (11).

2. A puncture treatment instrument for endoscope (1) comprising:
a concave portion (13) with an opening on a distal end side and a bottom surface on a proximal end side;
a holding portion (11) adjacent to a proximal end of the concave portion (13), the holding portion comprising a needle insertion path that allows advancement and retraction of a needle; and
a puncture needle (2) with a full length longer than a distance from a proximal end of the holding portion (11) to a distal end of the concave portion (13);
**characterized by** further comprising:
a string portion linking (24) a predetermined position of the puncture needle (2) and the holding portion (11), a length of the string portion in a tense state being a length such that a distal end of the puncture needle (2) is arranged in the concave portion (13) or in the holding portion (11).

3. The puncture treatment instrument for endoscope according to claim 1 or 2, wherein
the concave portion (13) is made of an elastic material, and a projected part (111) of an endoscope (100) can be fitted into the opening.

## Patentansprüche

1. Punktionsbehandlungsinstrument für ein Endoskop (1), umfassend:
einen konkaven Abschnitt (13) in konkaver Form mit einer Öffnung auf einer Seite des distalen Endes und einer Unterfläche (11a) auf der Seite des proximalen Endes, wobei eine Tiefe des konkaven Abschnitts von einem distalen Ende zur Unterfläche (11a) ein erster Abstand ist;
einen Halteabschnitt (11) mit einer Seite des distalen Endes neben einem proximalen Ende des konkaven Abschnitts (13), wobei der Halteabschnitt aus einem elastischen Material gefertigt ist, das das Einführen einer Nadel ermöglicht; und
eine Punktionsnadel (2), deren distales Ende in den Halteabschnitt (11) gesteckt ist und deren proximales Ende aus dem Halteabschnitt (11) herausragt, wobei eine volle Länge der Punktiernadel länger als ein Abstand von einem proximalen Ende des Halteabschnitts (11) zu einem distalen Ende des konkaven Abschnitts (13) ist;
**dadurch gekennzeichnet, dass** es ferner umfasst:
einen Widerhaken (4), der innerhalb des ersten Abstands vom distalen Ende auf einer Oberfläche der Punktionsnadel (2) angeordnet ist, wobei der Widerhaken das Zurückziehen der Punktionsnadel (2) stoppt, indem er von der Unterfläche (11a) des konkaven Abschnitts (13) erfasst wird, wenn die Punktionsnadel (2) zurückgezogen wird, nachdem die Punktionsnadel vorgeschoben wurde, um sich von dem Halteabschnitt (11) in den konkaven Abschnitt (13) zu erstrecken.

2. Punktionsbehandlungsinstrument für ein Endoskop (1), umfassend:
einen konkaven Abschnitt (13) mit einer Öffnung auf einer Seite des distalen Endes und einer Unterfläche auf einer Seite des proximalen Endes;
einen Halteabschnitt (11) neben einem proximalen Ende des konkaven Abschnitts (13), wobei der Halteabschnitt einen Nadeleinführpfad umfasst, der das Vorschieben und Zurückziehen einer Nadel ermöglicht; und
eine Punktionsnadel (2), deren volle Länge länger als ein Abstand von einem proximalen Ende des Halteabschnitts (11) zu einem distalen Ende des konkaven Abschnitts (13) ist;
**dadurch gekennzeichnet, dass** es ferner umfasst:
einen Schnurabschnitt, der eine vorgegebene Position der Punktionsnadel (2) mit der Halteposition (11) verbindet (24), wobei eine Länge des Schnurabschnitts in einem angespannten Zustand eine solche Länge aufweist, dass ein distales Ende der Punktionsnadel (2) im konkaven Abschnitt (13) oder im Halteabschnitt (11) angeordnet ist.

3. Punktionsbehandlungsinstrument für ein Endoskop nach Anspruch 1 oder 2, wobei der konkave Abschnitt (13) aus einem elastischen Material gefertigt ist und ein hervorstehender Abschnitt (111) eines Endoskops (100) in die Öffnung eingepasst werden kann.

## Revendications

1. Instrument de traitement de ponction pour endoscope (1) comprenant :
une partie concave (13) dans une forme concave avec une ouverture sur un côté extrémité distale et une surface de fond (11a) sur un côté extrémité proximale, une profondeur de la partie concave allant d'une extrémité distale à la surface de fond (11a) étant une première distance ;
une partie de maintien (11) avec un côté extrémité distale adjacent à une extrémité proximale de la partie concave (13), la partie de maintien étant faite d'un matériau élastique qui permet une insertion d'une aiguille ; et
une aiguille de ponction (2) avec une extrémité distale immobilisée dans la partie de maintien (11) et une extrémité proximale exposée à partir de la partie de maintien (11), une longueur totale de l'aiguille de ponction étant plus longue qu'une distance allant d'une extrémité proximale de la partie de maintien (11) à une extrémité distale de la partie concave (13) ;
**caractérisé par le fait qu'**il comprend en outre :
un ardillon (4) formé dans les limites de la première distance à partir de l'extrémité distale sur une surface de l'aiguille de ponction (2), l'ardillon arrêtant la rétractation de l'aiguille de ponction (2) en étant capturé par la surface de fond (11a) de la partie concave (13) lorsque l'aiguille de ponction (2) est rétractée après que l'aiguille de ponction a été avancée pour se projeter dans la partie concave (13) à partir de la partie de maintien (11).

2. Instrument de traitement de ponction pour endoscope (1) comprenant :
une partie concave (13) avec une ouverture sur un côté extrémité distale et une surface de fond sur un côté extrémité proximale ;
une partie de maintien (11) adjacente à une extrémité proximale de la partie concave (13), la partie de maintien comprenant un trajet d'insertion d'aiguille qui permet une avancée et une rétractation d'une aiguille ; et
une aiguille de ponction (2) avec une longueur totale plus longue qu'une distance allant d'une extrémité proximale de la partie de maintien (11) à une extrémité distale de la partie concave (13) ;
**caractérisé par le fait qu'**il comprend en outre :
une partie cordon (24) reliant une position prédéterminée de l'aiguille de ponction (2) et de la partie de maintien (11), une longueur de la partie cordon dans un état tendu étant une longueur telle qu'une extrémité distale de l'aiguille de ponction (2) est disposée dans la partie concave (13) ou dans la partie de maintien (11).

3. Instrument de traitement de ponction pour endoscope selon la revendication 1 ou 2, dans lequel
la partie concave (13) est faite d'un matériau élastique, et une partie en saillie (111) d'un endoscope (100) peut être montée dans l'ouverture.
